# FASCICULE DE BREVET EUROPEEN

(11) **EP 3 840 790 B1**
(45) Date de publication et mention de la délivrance du brevet: **21.06.2023**
(21) Numéro de dépôt: 18765044.5
(22) Date de dépôt: 23.08.2018
(51) Int. Cl.: A61L 27/36, B01L 1/04, C12M 1/00, C12M 3/00, C12M 1/36

(54) **PROCEDE AUTOMATISE DE TRAITEMENT D'UN TISSU BIOLOGIQUE POUR PRODUIRE UNE MATRICE TISSULAIRE**
AUTOMATISIERTES VERFAHREN ZUR BEHANDLUNG EINES BIOLOGISCHEN GEWEBES ZUR HERSTELLUNG EINER GEWEBEMATRIX
AUTOMATED METHOD FOR TREATING A BIOLOGICAL TISSUE IN ORDER TO PRODUCE A TISSUE MATRIX

(43) Date de publication de la demande: 30.06.2021
(73) Titulaire: Theracell Consulting SPRL, 1380 Lasne (BE)
(72) Inventeur: DUFRANE, Denis, 1380 Lasne (BE)
(74) Mandataire: Willnegger, Eva
(86) Numéro de dépôt international: PCT/EP2018/072741
(87) Numéro de publication internationale: WO 2020/038578

(56) Documents cités:
- WO-A1-2017/027481
- WO-A1-2018/122039
- WO-A2-2010/037192
- FR-A1- 3 016 311

## Description

La présente demande concerne le domaine de la production de tissus humains ou animaux, en vue de leur utilisation pour des greffes (allogreffes ou xénogreffes).

Une allogreffe implique un donneur et un receveur humain. Une autogreffe implique un seul être humain.

Une xénogreffe implique un donneur animal et un receveur humain.

Un nombre grandissant de troubles musculo-squelettiques humains nécessitent des traitements par reconstruction ou substitution osseuse, aussi bien dans le domaine dentaire (reconstruction maxillo-faciale par implant dentaire), qu'en cancérologie (suite à l'ablation de tumeurs osseuses) ou en chirurgie orthopédique (suite à une fracture ou pour la fusion de vertèbres).

Actuellement, la reconstruction osseuse se fait par autogreffe, généralement par ponction d'os au niveau de la crête iliaque de la personne qui nécessite le traitement. Cependant, en plus d'être douloureuse, cette ponction n'est pas réversible. En effet, l'os iliaque ne se régénère pas, et la quantité de tissu osseux disponible est donc limitée. L'autogreffe nécessite, en outre, une double intervention chirurgicale sur un même individu, ce qui a également un impact non négligeable sur la récupération postopératoire.

Une autre approche est l'utilisation de substituts osseux, largement accessibles et peu coûteux, comme par exemple des prothèses en céramique, corail ou verre. Malheureusement, du fait de la faible réglementation sur ce marché, de nombreux problèmes de fiabilité, de compatibilité de matériaux, voire même de toxicité sont à l'origine de nombreux problèmes post-opératoires et le personnel médical est de plus en plus méfiant vis-à-vis de ces substituts.

L'allogreffe est également pratiquée. En pratique, du tissu osseux est enlevé à un individu, par exemple une tête fémorale ôtée lors de la pose d'une prothèse de hanche. Ce tissu contient de la matrice osseuse ainsi que des cellules du donneur. Afin d'éliminer le risque de rejet, ou réponse inflammatoire, lors de la transplantation, il est nécessaire de traiter le tissu afin de le nettoyer de toutes traces « génétiques » du donneur, c'est-à-dire d'en éliminer les traces de cellules, de sang ou de graisse.

La méthode de nettoyage ou de « décellularisation » du tissu osseux pour donner une matrice osseuse decellularisée est décrite dans Dufrane et al., Biomaterials. 2002 Jul;23(14):2979-88 et Dufrane et al. Eur Cell Mater. 2001 Jan 10;1:52-8; discussion 58.

Les principales étapes de cette méthode de nettoyage sont :
- Centrifugation du tissu prélevé du donneur afin d'en éliminer le sang et la graisse ;
- Découpe du tissu, généralement en cubes de taille variable ;
- Traitement dans une ou plusieurs solutions, afin d'en éliminer les traces de cellules, et d'inactiver les virus et/ou bactéries.

Après nettoyage, on procède à une lyophilisation, afin d'obtenir de la matrice osseuse déminéralisée stable et à un emballage.

Bien que l'allogreffe soit à ce jour la technique de reconstruction osseuse la plus fiable, l'accès des patients à cette technique est fortement restreint par la méthode et les coûts de production de la matrice osseuse décellularisée. La demanderesse a mis au point un procédé automatisé pour réaliser ces étapes dans une enceinte stérile classée, procédé décrit dans WO2018122039. Ce procédé d'obtention d'une matrice tissulaire pour allogreffe ou xénogreffe, comprend, après récupération d'un tissu biologique, les étapes selon lesquelles:
- on classe ledit tissu,
- on découpe éventuellement le tissu,
- on traite ledit tissu et
- on conditionne la matrice tissulaire obtenue,
toutes ces étapes étant mises en oeuvre de façon automatisée à l'intérieur d'un même réacteur « classé ». Les principaux avantages de ce procédé sont d'assurer la traçabilité des lots quant à l'origine du tissu, en évitant notamment les possibles erreurs humaines, et de diminuer considérablement le coût de production de matrices tissulaires.

Si les étapes de classement, découpe et conditionnement sont des étapes plutôt courtes, c'est-à-dire ne durant que quelques minutes, l'étape de traitement des tissus peut durer plusieurs heures et est donc susceptible de limiter la productivité de l'automate mettant en oeuvre le procédé.

En effet, un traitement de tissu implique l'agitation du tissu dans plusieurs bains successifs. Il est donc nécessaire, une fois le tissu découpé en morceaux, d'introduire les morceaux dans un premier bain, de l'y agiter durant un certain temps, de retirer le tissu du bain et de l'introduire dans le bain suivant, de l'y agiter à nouveau durant un temps déterminé, et ainsi de suite jusqu'au traitement complet, dont le nombre de bains successifs peut varier selon la nature du tissu. De plus, chaque tissu doit être traité de façon individuelle de façon à éviter les contaminations croisées.

Pour maintenir une cadence élevée de traitement de tissu, il est alors nécessaire de mettre en oeuvre, en parallèle, plusieurs (voire un grand nombre) de lignes de traitement comprenant chacune une série de récipients contenant les solutions dans lesquelles les morceaux de tissus doivent être successivement plongés. Cela implique d'avoir à disposition un grand nombre de récipients de traitement. En particulier, si on considère que ces récipients doivent être remplacés entre chaque lot de tissu, il faut introduire un grand stock de ces récipients à l'intérieur de l'enceinte classée. Cette façon de procéder n'est ainsi ni écologique, ni bon marché.

Le document WO 2018/122039 A1 décrit un procédé automatisé de traitement d'un tissu biologique pour produire une matrice tissulaire pour allogreffe ou xénogreffe. Ce Document ne décrit pas que toutes les étapes de traitement du tissu sont réalisées au sein d'un unique récipient-réacteur", en effet, dans cet art antérieur, les paniers sont déplacés d'une solution à l'autre de façon manuelle.

Ces problèmes de production ne sont pas limités aux tissus osseux, mais sont également rencontrés pour d'autres tissus biologiques utilisés en allogreffe, comme la peau, les tendons, la vessie ou le tissu adipeux. Ces mêmes problèmes sont également rencontrés pour la production des thérapies cellulaires ou lors du traitement de cellules dérivées de tissus.

Afin d'améliorer l'efficacité de traitement automatisé des tissus, la demanderesse a mis au point un procédé et un support résolvant les problèmes cités ci-dessus.

### Solution de l'invention

La présente invention propose, à cet effet, un procédé automatisé de traitement d'un tissu biologique pour produire une matrice tissulaire pour allogreffe ou xénogreffe, selon lequel la séquence des étapes suivantes:
a) on introduit ledit tissu dans une solution de traitement,
b) on procède au traitement du tissu dans la solution, sous agitation,
c) on sort ledit tissu de la solution de traitement,
d) on vide le récipient réacteur,

est répétée jusqu'à la fin du traitement,
caractérisé par le fait que toutes les étapes de toutes les séquences sont mises en oeuvre de façon automatisée dans une enceinte « classée » et à l'intérieur d'un unique récipient-réacteur, en changeant la solution de traitement dans le récipient-réacteur entre deux séquences.

Par tissu biologique, on entend également des morceaux de tissus biologiques, c'est-à-dire un tissu biologique ayant déjà subi une étape de découpe. Le procédé de l'invention, mis en oeuvre dans une enceinte « classée », s'effectue dans des conditions stériles et correspondant à des normes bien précises.

Pour mettre en oeuvre le procédé de l'invention, il est également proposé un récipient-réacteur comprenant :
- un conteneur agencé pour contenir une solution de traitement de tissu biologique dans lequel est introduit
- un récepteur de tissu biologique et
- des moyens d'agitation,
caractérisé par le fait que le récepteur est amovible.

Avantageusement, les moyens d'agitation sont associé au récepteur, ce qui permet de ne fabriquer qu'un élément ayant les deux fonctions d'agitation et de support de tissu biologique.

Ainsi, le tissus biologique sur son récepteur peut être introduit dans le récipient-réacteur contenant une solution de traitement pour procéder à l'étape a) du procédé de l'invention ; les moyens d'agitation permettent la mise en oeuvre de l'étape b) de traitement sous agitation ; le récepteur étant amovible, il permet d'extraire le tissu biologique de la solution selon l'étape c); le récipient peut être vidé de sa solution selon l'étape d) et la séquence d'étapes peut être renouvelée. Un récipient-réacteur unique permet de procéder à toutes les étapes du traitement. Il permet ainsi de réduire l'encombrement dans l'enceinte de traitement. Un grand nombre de lots peuvent ainsi être traités en même temps dans l'enceinte classée, atténuant ainsi l'engorgement résultant de la relativement longue durée du traitement par rapport aux autres étapes d'introduction, découpe, conditionnement....

Le récepteur amovible est agencé de façon à être au moins en partie perméable aux liquides, c'est-à-dire ajouré de façon à servir de support au tissu biologique et à lui permettre de s'immerger dans la solution de traitement à l'étape a) et de l'égoutter lorsqu'il est extrait du conteneur à l'étape c).

Chaque lot de tissu étant conservé dans un même récipient tout au long du traitement, sa traçabilité est assurée, ainsi que l'absence de contamination croisée.

Avantageusement, le procédé de traitement de l'invention peut être mis en oeuvre plus spécifiquement dans le procédé d'obtention d'une matrice tissulaire pour allogreffe ou xénogreffe, de façon automatisée à l'intérieur d'un unique réacteur « classé », selon lequel, après récupération d'un tissu biologique :
- on classe ledit tissu,
- on découpe ledit tissu,
- on traite ledit tissu et
- on conditionne la matrice tissulaire obtenue,

dans lequel pour traiter le tissu, répète, jusqu'à la fin du traitement, la séquence des étapes suivantes :
   a) on introduit ledit tissu dans une solution de traitement,
   b) on procède au traitement du tissu dans la solution, sous agitation,
   c) on sort ledit tissu de la solution de traitement,
   d) on vide le récipient réacteur,
toutes les étapes de toutes les séquences du traitement étant mises en oeuvre à l'intérieur d'un unique récipient-réacteur en changeant la solution de traitement dans le récipient-réacteur entre deux séquences.

Pour la mise en oeuvre de ce procédé, le récipient réacteur de l'invention est avantageusement compris dans un kit comprenant un plateau supportant :
- le récipient-réacteur de l'invention, et
- un réceptacle de tissu à traiter.

Le kit peut en outre comprendre
- des doigts de préhension de tissu biologique, agencés pour être montés sur un automate de découpe de tissu et/ou
- des doigts de préhension de tissu biologique, agencés pour être montés sur un automate de déplacement du tissu.

Par automate, on entend un moyen automatisé, comme un robot ou un bras robotisé.

Le réceptacle du tissu à traiter comprend avantageusement des moyens de positionnement du tissu. Cela permet, dès l'introduction du tissu dans le réacteur classé, qu'il soit positionné de façon à être pris en charge de façon optimale par les automates assurant les différentes étapes du procédé d'obtention de matrice tissulaire.

Les doigts de préhension de tissu biologique, agencés pour être montés sur l'automate de découpe de tissu doivent être capable de conserver le tissu durant l'étape de découpe qui peut faire intervenir un jet d'eau à haute pression.

Il faut comprendre ainsi qu'un kit correspond à un lot de tissu à traiter. Il accompagne ce tissu depuis son introduction dans l'enceinte jusqu'au conditionnement final, les éléments du kit étant utilisés puis éventuellement jetés après utilisation. Ce kit est donc de préférence jetable.

Le kit peut comprendre également le ou les conteneurs finaux dans le(s)quel(s) la matrice tissulaire, éventuellement en morceaux, sera conditionnée et emballée.

La solution de traitement du tissu peut être une solution de traitement chimique ou de traitement biologique. Le type de solution à utiliser en fonction du tissu à traiter est connu de l'homme du métier.

Un traitement peut être un traitement chimique comprenant l'exposition du tissu biologique à des agents ou réactifs chimiques, par exemple l'agitation du tissu dans une solution contenant un agent antibactérien, un solvant organique ou une solution dont le pH est ajusté acide ou basique, par ajout de composés ioniques.

Un traitement biologique fait référence à l'application d'agents biologiques au tissu concerné, comme par exemple des facteurs de croissance ou des protéines induisant une différenciation cellulaire.

Le tissu biologique peut subir plusieurs traitements consécutifs ayant des finalités différentes, comme par exemple le dégraissage ou l'inactivation des bactéries ou des virus ou du prion.

Il est souligné ici que le procédé automatisé de traitement d'un tissu biologique, le procédé d'obtention d'une matrice tissulaire, le récipient-réacteur et le kit sont liés par le même concept inventif qui est la réduction du volume des équipements à l'intérieur de l'enceinte et la réduction de l'espace-temps nécessaire au traitement pour permettre à la ligne de production de matrice tissulaire de fonctionner en continu, malgré des étapes de durées très différentes.

L'invention sera mieux comprise à l'aide de la description suivante de plusieurs mises en oeuvre de l'invention, en référence au dessin en annexe, sur lequel :
- la figure 1 est une illustration schématique d'une enceinte stérile pour la mise en oeuvre de l'invention,
- la figure 2 représente, en perspective, l'intérieur d'une enceinte stérile pour la mise en oeuvre du procédé de l'invention,
- la figure 3 est une illustration schématique du procédé de l'invention,
- les figures 4 et 5 sont des illustrations en perspective d'un récipient-réacteur selon l'invention, dans deux positions différentes,
- la figure 6 est une illustration en perspective d'un kit selon l'invention,
- la figure 7 est une illustration en perspective d'un doigt de préhension du kit de la figure 6 et
- la figure 8 est une illustration en perspective d'un autre doigt de préhension du kit de la figure 7.

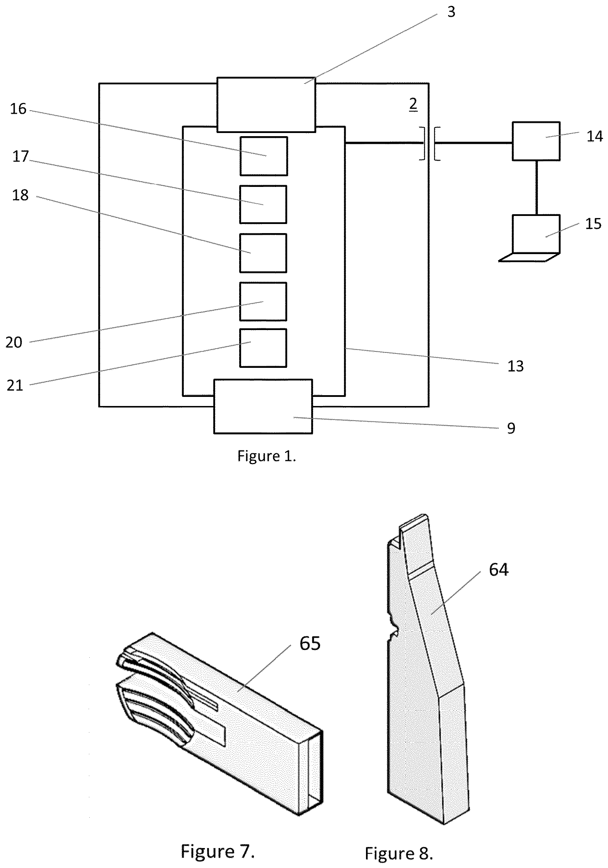

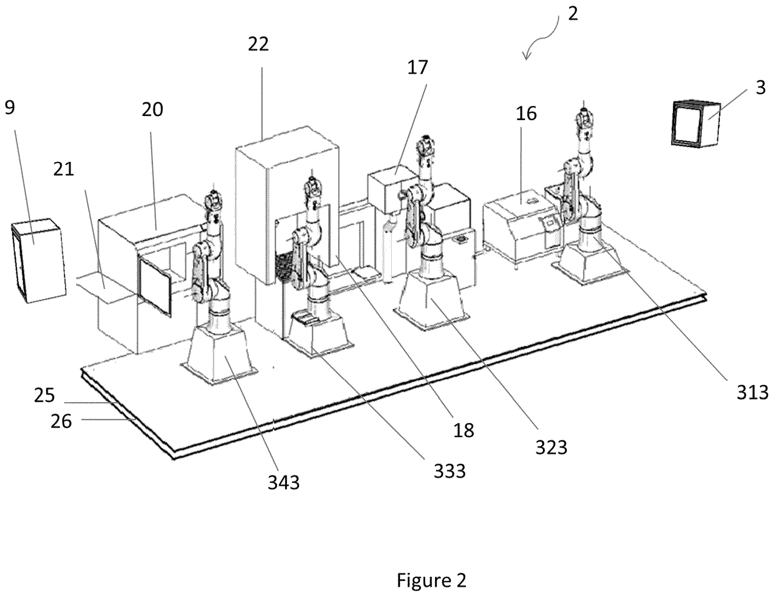

En référence aux figures 1 et 2 illustrant le procédé et le réacteur décrits dans WO2018122039, à l'intérieur du réacteur 2, une série 13 d'automates 313, 323, 333 et 343, coopérant entre eux, est agencé pour permettre la mise en oeuvre des étapes du procédé. Cette série d'automates 13 est avantageusement pilotée par un système informatique 14 auquel des instructions auront éventuellement été données par un opérateur, au moyen d'un poste de pilotage 15, ici un ordinateur.

Le réacteur 2 comprend un sas 3 d'introduction et de classement de tissus biologiques, prélevés sur un donneur et un sas 9 de sortie de matrice tissulaire conditionnée, chaque sas étant ici placé à une extrémité du réacteur. Entre les deux sas sont ici placés, côte à côte, cinq équipements: une centrifugeuse 16, une unité 17 de découpe, une station 18 de traitement chimique, placée sous une hotte 22, un lyophilisateur 20 et une station d'emballage 21, devant lesquels sont positionnés respectivement les automates, ici quatre bras robotiques articulés 313, 323, 333 et 343. Tous les éléments décrits ci-dessus sont placés sur un plancher 25 sous lequel est prévu un espace 26.

Les éléments du réacteur 2 ayant été décrits, l'articulation des éléments entre eux pour la mise en oeuvre du procédé de l'invention va maintenant être détaillée pour un tissu qui peut, par exemple, être une tête fémorale, prélevée sur un individu donneur, lors de la pose d'une prothèse de hanche. Cette tête fémorale brute, pour pouvoir servir à la fabrication de matrice osseuse en vue de son utilisation pour une reconstruction osseuse chez un autre patient, doit subir un certain nombre de transformations, dans un environnement stérile contrôlé.

Après avoir ouvert la porte externe du sas 3, un opérateur dépose dans ce sas un tissu biologique à traiter, dans un réceptacle approprié. Le flux d'air à l'intérieur du sas est tel qu'aucune contamination extérieure ne peut pénétrer dans le sas, jusqu'à ce que la porte externe soit refermée.

Le procédé automatisé d'obtention de matrice cellulaire débute par l'ouverture de la porte intérieure (au réacteur 2) du sas 3. Le bras robotique 313 vient saisir le réceptacle contenant le tissu biologique dans le sas 3 et le dépose dans une cavité de la centrifugeuse 16.

La centrifugation permet de séparer la matrice osseuse de résidus indésirables, tels que la moelle, le sang ou des résidus graisseux. La tête fémorale, ainsi nettoyée, est ensuite saisie par l'automate de découpe 323 qui la déplace vers l'unité de découpe 17, configurée pour produire des morceaux d'os à un calibre prédéfini. Ici, une découpe par jet à haute pression d'eau, non abrasif est mise en oeuvre afin d'une part de couper très précisément et très nettement, sans élévation de température pouvant endommager les tissus, et, d'autre part, de ne pas contaminer les lots entre eux.

En pratique, le bras 323 maintenant le tissu biologique à découper est mobile selon plusieurs axes simultanément et vient positionner et déplacer le tissu à découper sous le jet, selon n'importe quelle orientation prédéfinie. Le bras articulé étant mobile selon plusieurs axes, il est ainsi possible d'obtenir n'importe quelle géométrie de découpe, ce qui permet d'optimiser la découpe afin de limiter les déchets et d'obtenir un rendement plus élevé de matrice tissulaire en fin de procédé.

Ces morceaux sont ensuite récupérés par le bras 333 et introduits dans une zone de traitement 18, où ils vont subir plusieurs séquences successives d'étapes d'immersion et d'agitation dans des solutions de traitement, éventuellement complétées par une ou plusieurs étapes de rinçage. Cette étape de traitement va être décrite en détails plus bas.

Le bras robotique 333 récupère les morceaux de tissus biologique traités un par un pour les disposer chacun dans récipient de conditionnement, comme par exemple un tube pré-scellé, c'est-à-dire un tube dont les capuchons sont déjà disposés sans toutefois être scellés, puis introduit chaque tube dans le lyophilisateur 20. A la fin de la lyophilisation, avant réouverture du lyophilisateur, les tubes sont scellés, par exemple grâce à une plaque qui vient appuyer sur les capuchons afin de sceller les tubes. Chaque tube comprend ainsi un morceau de matrice tissulaire, prêt à être utilisé pour une greffe.

Le bras 343 va ensuite terminer le conditionnement des tubes, en y déposant par exemple une étiquette comprenant toutes les informations nécessaires à la traçabilité du lot, puis déposer les tubes de matrice tissulaire conditionnée dans le sas 9 de sortie afin qu'ils soient récupérés par un opérateur.

Les actions réalisées ici par les bras robotiques 333 et 343 pourraient être réalisés par un seul bras, ou d'autres bras automatisés pourraient être ajoutés. Notons ici que l'articulation précise des différentes tâches effectuées par les bras robotisés est très flexible et tout autre arrangement pertinent, avec plus ou moins de bras, peut être envisagé.

La lyophilisation permet de sécher la matrice tissulaire jusqu'à ce qu'elle contienne, par exemple, moins de 5% d'humidité, et puisse être emballée sous vide ou sous atmosphère protectrice dans une unité d'emballage 21. Cela assure à la matrice osseuse une stabilité optimale permettant sa conservation à température ambiante pendant plusieurs mois. Il est envisageable d'inclure une étape d'étiquetage des emballages afin d'assurer la traçabilité des lots produits. La matrice tissulaire emballée peut alors être extraite du réacteur 2 par le sas de sortie 9, similaire au sas d'entrée 3. Il est même possible de configurer le réacteur 2 pour qu'il n'ait qu'un seul sas d'entrée et de sortie.

Le poste de commande, de pilotage 15 comprend le système informatique 14 sur lequel est renseigné le procédé de fabrication du tissu emballé. Le système informatique 14 pilote la série 13 d'automates dans le réacteur 2. Certains paramètres peuvent devoir être renseignés par l'opérateur, comme par exemple la nature du tissu, si l'automate peut gérer la production de plusieurs types de tissus, les durées des traitements chimiques ou le pourcentage d'humidité désiré dans la matrice tissulaire emballée.

Le procédé de fabrication décrit ci-dessus permet donc d'introduire un tissu biologique « brut » dans un réacteur et de récupérer en sortie un tissu biologique décontaminé et prêt à être utilisé pour une greffe. Il n'y a dans ce procédé qu'une seule étape de classification, à l'entrée du tissu 1 dans le réacteur 2. La continuité de la classification est assurée par l'espace restreint et contrôlé du réacteur dont le tissu ne sort pas avant la fin de la production. Aucun opérateur n'ayant à intervenir durant le déroulement du procédé, les erreurs de manipulation sont exclues, la traçabilité est assurée, les besoins en espace et en manutention sont réduits au strict minimum.

L'étape de traitement est néanmoins longue comparée aux autres étapes que doit subir le tissu biologique. Cette étape doit donc être optimisée et intégrée dans le procédé global de production de matrice tissulaire de façon à ne pas en limiter l'efficacité et d'optimiser le temps d'utilisation de chaque élément/appareil contenu dans l'enceinte.

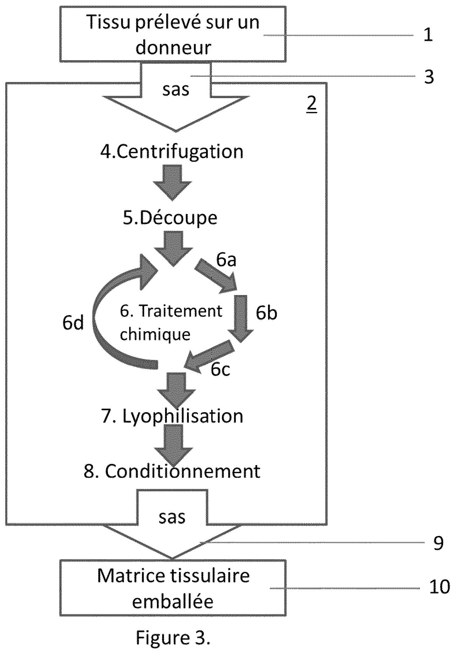

En référence à la figure 3, les étapes du procédé d'obtention de matrice tissulaire 10 sont la centrifugation 4 du tissu 1, la découpe 5 de ce tissu, le traitement 6, la lyophilisation 7 et le conditionnement, toutes conduites dans une enceinte stérile 2 pourvue d'un sas 3 d'introduction de tissu et un sas 9 de sortie de la matrice tissulaire.

L'étape 6 de traitement comprend plusieurs sous-étapes. Dans l'étape 6a, le tissu en morceaux est introduit dans une solution de traitement à l'intérieur d'un récipient réacteur, puis à l'étape 6b, le traitement des morceaux de tissu dans la solution a lieu, c'est-à-dire que le tissu est agité pendant une durée prédéterminée dans la solution de traitement. Le tissu est ensuite, à l'étape 6c sorti de la solution de traitement, et à l'étape 6d, le récipient réacteur est vidé de la solution qu'il contient.

La séquence des étapes 6a à d est renouvelée jusqu'à la fin du traitement, c'est-à-dire qu'une nouvelle solution de traitement, de même composition ou de composition différente, est introduite dans le même récipient réacteur qu'utilisé dans la première séquence, les morceaux de tissus y sont introduits, agités le temps nécessaire puis en sont ressortis.

Lorsque toutes les séquences nécessaires au traitement ont été réalisées, les morceaux de tissus peuvent alors être lyophilisés et conditionnés.

On comprend ainsi qu'un lot de tissu biologique est traité dans un récipient dit « récipient-réacteur ». Ce récipient-réacteur est fabriqué de façon à pouvoir contenir successivement une variété de solutions de traitement, généralement de solutions de traitement chimiques. Il n'y a pas de multiples récipients contenant chacun un type de solution dans lesquels le tissu serait successivement plongé. Il en résulte un gain de place considérable dans l'enceinte stérile.

Cela permet en outre d'initier en parallèle, les uns après les autres, le traitement de plusieurs lots de tissu, en limitant le nombre de récipients à stocker dans l'espace de traitement dans l'enceinte stérile.

Par exemple, dès que la découpe d'un premier lot est terminée, les morceaux sont introduits dans un premier récipient-réacteur et plusieurs séquences successives d'étapes de traitement 6a à d dans plusieurs solutions de traitement, successivement introduites dans le premier récipient-réacteur, vont commencer. Pendant ce temps, un deuxième lot de tissu peut être découpé en morceaux, qui sont ensuite introduits dans un deuxième récipient-réacteur et les séquences successives d'étapes de traitement 6a à d sont également initiées dans le deuxième récipient réacteur. Ainsi, les traitements de multiples lots peuvent être initiés successivement et en parallèle, c'est-à-dire dans le même espace, mais décalés dans le temps. Dès que le traitement du premier lot est terminé, les morceaux de tissu du premier lot sont envoyés à l'étape de lyophilisation, libérant ainsi de l'espace pour débuter le traitement d'un nouveau lot, dans son récipient-réacteur. Idéalement, le nombre de lots qu'il est possible de traiter dans l'espace dédié au traitement de l'enceinte stérile est ajusté en fonction du temps nécessaire au traitement par rapport au temps nécessaire aux autres étapes, comme par exemple la découpe ou la lyophilisation. Les équipements installés dans l'enceinte stérile peuvent ainsi idéalement fonctionner en continu.

Pour procéder à un tel traitement, le récipient-réacteur doit présenter certaines caractéristiques essentielles. Il doit notamment permettre qu'une solution de traitement y soit introduite, que les morceaux de tissus y soient immergés, qu'ils puissent y être agités, puis que les morceaux de tissus en soient retirés et que la solution soit vidée du récipient.

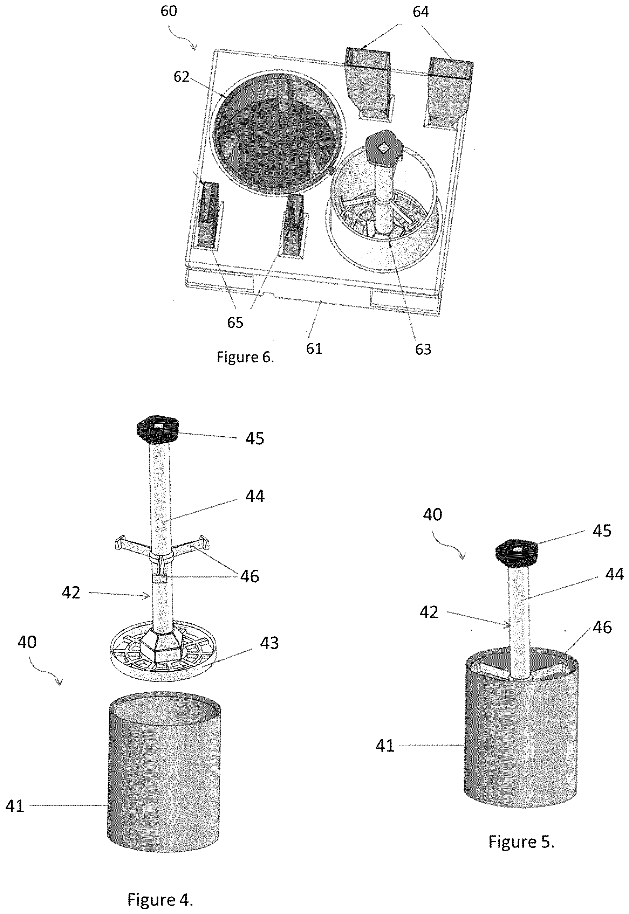

En référence aux figures 4 et 5, un tel récipient réacteur 40 comprend un conteneur 41, ici cylindrique, et un récepteur 42 de tissu biologique. Le récepteur 42 comprend ici une base 43 circulaire ajourée, dont le diamètre est légèrement inférieure au diamètre du conteneur 41. La base 43 est placée au bout d'une tige 44, dont l'autre extrémité est terminée par une noix d'assemblage 45. A niveau du milieu de la tige, trois pales 46 ou hélices sont disposées radialement, formant un angle de 130° entre elles. Le récepteur 42 est dissocié du conteneur 41 mais est conformé pour s'y insérer. Lorsque le récepteur 42 est inséré dans le conteneur 41, seule une partie de la tige 44 et la noix de couplage 45 dépassent du conteneur 41.

En pratique, pour le traitement dont il s'agit ici et en référence à l'enceinte de la figure 2, le bras 333 ayant récupéré les morceaux de tissus découpés, il les dépose sur la base 43 du récipient-réacteur 40, soit lorsque le récepteur est inséré dans le conteneur, soit lorsqu'il en est sorti, ou partiellement sorti. A ce stade, le récipient-réacteur peut être déjà présent dans la zone de traitement 18 ou alors y être introduit seulement lorsque les morceaux de tissus ont déjà été déposés sur le récepteur 42.

Une première solution de traitement est introduite dans le conteneur 41, soit lorsque le récepteur 42 supportant le tissu y est déjà introduit, soit, et de préférence, avant son introduction. Dans ce dernier cas, le récepteur 42 supportant le tissu est alors plongé dans la solution de traitement contenue dans le conteneur 41, permettant au tissu d'y être immergé.

Le récepteur 42 est alors entraîné en rotation, par l'intermédiaire de la noix de couplage 45. La tige 42, la base 43 et les pales 46 sont alors animées d'un mouvement rotatif, les pales 46 étant pourvues d'ailettes à leurs extrémités, elles permettent l'homogénéisation de la solution de traitement.

La noix de couplage 45 permet à des moyens robotisés de la zone de traitement 18 de saisir le récepteur 42 pour lui impartir le mouvement de rotation nécessaire à l'agitation. Elle peut également permettre aux mêmes moyens robotisées, ou à d'autres, d'impartir au récepteur 42 un mouvement de translation verticale pour introduire le récepteur 42 dans le conteneur 41 ou l'en sortir.

Lorsque le tissu a été suffisamment agité, des moyens robotisés soulèvent le récepteur 42 hors du conteneur 41. La configuration ajourée de la base 43 permet d'égoutter les morceaux de tissus. Le conteneur 41 peut alors être vidé de la solution de traitement qu'il contient. La solution peut par exemple être renversée par inclinaison ou retournement du conteneur 41, elle peut être aspirée par des moyens automatisés prévus dans la zone de traitement, ou encore un robinet d'évacuation peut être prévu dans le fond du conteneur qui serait actionné par des moyens automatisés prévus dans la zone de traitement.

Si une autre séquence d'étapes de traitement est prévue, le conteneur 41 peut éventuellement être rincé avant d'y introduire une autre solution de traitement, afin d'éviter des réactions indésirables.

Alternativement, il est possible qu'une séquence d'étapes de traitement soit une séquence de rinçage, auquel cas une solution de rinçage est introduite dans le conteneur 41, le récepteur 42 supportant les morceaux de tissu est réintroduit dans le conteneur 41 et l'agitation est mise en route pendant une durée déterminée avant de retirer à nouveau le récepteur 42 du conteneur 41 et d'en vider la solution de rinçage.

Une nouvelle séquence de traitement peut alors commencer, dans une autre solution de traitement.

Si le traitement est fini, les morceaux de tissus égouttés sont alors pris en charge par le bras robotique 333 pour la lyophilisation. Le récipient-réacteur 40 peut alors être jeté, ou mis de côté pour être recyclé. Ainsi, un récipient réacteur n'est utilisé qu'une seule fois, pour un seul lot de tissu. Cela permet d'éviter de mélanger des tissus de lots différents et/ou de transmettre des contaminations d'un lot à un autre.

Il peut être envisagé d'organiser la zone de traitement 18 comme un carrousel ou les récipients-réacteurs des lots arrivant se déplacent progressivement le long d'une « chaine » où peuvent être réalisées les différentes étapes des séquences successives, le lot de tissu dans son récipient-réacteur parcourant ainsi une sorte de boucle de traitement, de nouveaux récipient-réacteurs contenant de nouveaux lots de tissus à traiter pouvant être déposés en continu sur cette chaine/boucle.

Astucieusement, le récipient-réacteur 40 peut faire partie d'un kit comprenant des éléments matériels à usage uniques, destinés à accompagner le tissu biologique au cours des étapes qu'il doit subir avant d'obtenir une matrice tissulaire. Le kit permet notamment de faciliter l'introduction du tissu biologique via le sas 3, d'améliorer sa traçabilité et d'éviter les contaminations croisées entre lots de tissus.

En référence à la figure 6, un kit 60 comprend un plateau 61 supportant un réceptacle 62 de tissu, un récipient-réacteur 63, deux paires 64 et 65 de doigts de préhension de tissu biologique. Le récipient-réacteur 63 correspond ici au récipient-réacteur illustré sur les figures 4 et 5.

Le réceptacle 62 est ici agencé avec trois plots 66 destinés à y positionner une tête fémorale d'une façon particulière, pour son introduction dans l'enceinte stérile. Ainsi, la tête fémorale est optimalement positionnée pour le premier bras robotisé devant la prendre en charge.

Selon la nature du tissu dont on cherche à obtenir une matrice tissulaire, le réceptacle 62 peut être agencé différemment et de n'importe quelle manière adaptée.

Le réceptacle 62 peut être agencé pour pouvoir être introduit directement dans la centrifugeuse. Par exemple, un bras robotisé récupère le plateau 61 du kit 60, y prélève le réceptacle 62 contenant le tissu biologique, introduit le réceptacle dans la centrifugeuse, l'en ressort à la fin de la centrifugation et replace le réceptacle sur le plateau 61 avant de transmettre le kit 60 à l'étape suivante. Par exemple, le kit est déposé sur un rack intermédiaire en attendant sa prise en charge par le bras robotisé suivant, ou pour que le bras robotisé de découpe vienne juste y prélever le tissu.

Avantageusement, le réceptacle est agencé pour séparer le tissu des fluides expulsés lors d'une centrifugation. Il peut par exemple comprendre une grille supportant le tissu et le séparant du fond du réceptacle afin que les fluides expulsés du tissu lors de le centrifugation soient récupérés dans le fond, mais pas en contact avec le tissu.

Le bras de découpe, avant de saisir le tissu biologique, ici la tête fémorale va se munir de doigts de préhension, ici une paire 65, spécifiquement adaptés pour pouvoir soumettre le tissu biologique à un jet d'eau à haute pression, sans que ces doigts ne lâchent le tissu biologique sous l'effet de la pression. La conformation ou la matière utilisée pour ces doigts de préhension seront bien évidemment différents si le tissu est de la peau ou de l'os, un tissu mou ou un tissu dur. Ils peuvent par exemple comprendre des picots permettant de retenir le tissu lors de la découpe, ou des rainures, comme illustré sur la figure 7. Ces doigts comprennent ainsi des moyens pour agripper un tissu biologique.

Selon les bras robotisés utilisés, il peut y avoir plus de deux doigts.

A la fin de la découpe, les doigts sont retirés du bras robotiques pour être jetés, ou remis sur le plateau 61 du kit 60, ou encore mis de côté pour être recyclés. Les doigts de préhension agencés pour être montés sur le bras/l'automate de découpe sont ainsi à usage unique, tels des gants. Cela évite la contamination entre les différents lots de tissus. Cette caractéristique est particulièrement intéressante en combinaison avec une découpe à jet d'eau à haute pression, celle-ci évitant également les possibilités de contamination croisées qui sont inévitables quand on utilise des systèmes de découpes à lames ou à bandes, dont il est impossible de remplacer les parties coupantes entre deux lots.

L'étape de traitement est ensuite réalisée à l'aide du récipient-réacteur 63, comme cela a été détaillé plus haut.

L'autre paire 64 de doigts de préhension est destinée au(x) bras robotisé(s)/automate(s) devant prendre en charge les morceaux de tissus, notamment pour les déplacer, par exemple juste après la découpe ou après le traitement. Les doigts de préhension pour déplacement sont d'un design différent de la paire destinée à l'étape de découpe, et peut par exemple ressembler au doigt illustré sur la figure 8. Les mêmes doigts de préhension peuvent être utilisés par un ou plusieurs bras pour la prise en charge d'un même lot de tissu biologique. Comme pour la découpe, cela évite les contaminations entre lots.

Les doigts de préhension sont ici décrits comme faisant partie du kit. Selon la configuration de l'enceinte, les doigts peuvent être stockés à l'intérieur de l'enceinte, indépendamment du kit, et prélevés dans ce stock au fur et à mesure des besoins. Dans d'autres configurations, il peut être prévu que les doigts soit nettoyés et/ou désinfectés entre chaque lot à l'intérieur de l'enceinte.

On peut éventuellement prévoir que des tissus de natures différentes puissent être traités successivement dans l'enceinte stérile. Dans ce cas, chaque tissu est introduit dans un kit comprenant les éléments (réceptacle, doigts, récipient-réacteur) approprié à son traitement spécifique. Le kit suivant le tissu tout au long des étapes d'obtention de la matrice tissulaire, et fournissant les éléments qui lui sont spécifiques, l'équipement intérieur de l'enceinte stérile n'aurait ainsi pas besoin d'être modifié pour traiter successivement des tissus de nature différente. Il reste alors uniquement à introduire les paramètres informatiques gérant les étapes réalisées dans l'enceinte.

Les éléments du kit sont avantageusement tous marqués d'un identifiant de suivi du lot de tissu à traiter, comme par exemple un numéro, un code barre ou un code QR. Le kit pourrait également contenir d'autres éléments tels que le ou les emballage(s) des morceaux de matrice tissulaire finale.

Le plateau 61 du kit est avantageusement conformé pour être pris en charge par les équipements à l'intérieur de l'enceinte stérile, et/ou pour y être momentanément stocké sur un rack ou une étagère.

Le kit est avantageusement un kit jetable qui est mis au rebut et/ou décontaminé après usage pour être recyclé.

Bien que l'invention ait ici été décrite en prenant l'exemple de matrice osseuse obtenue à partir d'une tête fémorale, elle peut être appliquée à d'autres tissus biologiques, comme par exemple ceux issus d'autres sources osseuses ou des tissus mous comme le fascia lata.

## Revendications

1. Procédé automatisé de traitement d'un tissu biologique pour produire une matrice tissulaire pour allogreffe ou xénogreffe, selon lequel la séquence des étapes suivantes :
a) on introduit (6a) ledit tissu dans une solution de traitement,
b) on procède au traitement du tissu dans la solution, sous agitation (6b),
c) on sort (6c) ledit tissu de la solution de traitement,
d) on vide (6d) le récipient réacteur,
est répétée jusqu'à la fin du traitement,
**caractérisé par le fait que** toutes les étapes de toutes les séquences sont mises en oeuvre de façon automatisée dans une enceinte « classée » (2) et à l'intérieur d'un unique récipient-réacteur (40), en changeant la solution de traitement dans le récipient-réacteur entre deux séquences.

2. Procédé d'obtention d'une matrice tissulaire pour allogreffe ou xénogreffe, de façon automatisée à l'intérieur d'un unique réacteur « classé », selon lequel, après récupération d'un tissu biologique :
- on classe ledit tissu,
- on découpe (4) ledit tissu,
- on traite (6) ledit tissu et
- on conditionne (8) la matrice tissulaire obtenue,
dans lequel pour traiter le tissu, répète, jusqu'à la fin du traitement, la séquence des étapes suivantes :
a) on introduit (6a) ledit tissu dans une solution de traitement,
b) on procède au traitement du tissu dans la solution, sous agitation (6b),
c) on sort (6c) ledit tissu de la solution de traitement,
d) on vide (6d) le récipient réacteur,
toutes les étapes de toutes les séquences du traitement étant mises en oeuvre à l'intérieur d'un unique récipient-réacteur (40) en changeant la solution de traitement dans le récipient-réacteur (40) entre deux séquences.

3. Procédé selon la revendication 2, comprenant également les étapes de :
- on centrifuge (4) le tissu biologique, et/ou
- on lyophilise (7) la matrice tissulaire avant conditionnement.

4. Récipient-réacteur (40) pour la mise en oeuvre du procédé selon les revendications 1 à 3 comprenant :
- un conteneur (41) agencé pour contenir une solution de traitement de tissu biologique dans lequel est introduit
- un récepteur (42) de tissu biologique et
- des moyens d'agitation (46),
**caractérisé par le fait que** le récepteur est amovible.

5. Récipient-réacteur (40) selon la revendication 4, dans lequel le récepteur amovible comprend une base (43) ajourée de support de tissu biologique.

6. Récipient-réacteur selon l'une des revendications 4 et 5, dans lequel les moyens d'agitation (46) sont associés au récepteur (42).

7. Récipient-réacteur (40) selon les revendications 5 et 6, dans lequel les moyens d'agitation (46) comprennent au moins une pale (46) disposée radialement le long d'une tige (42) dont une extrémité est la base (43) ajourée de support de tissu biologique.

8. Récipient-réacteur (40) selon la revendication 7, dont le conteneur (41) est agencé pour contenir au moins la partie de la tige (42) sur laquelle est disposée la pale (46) et la base (43).

9. Kit (60) comprenant un plateau (61) supportant :
- le récipient-réacteur (40) selon l'une des revendications 4 à 8 et
- un réceptacle (62) de tissu à traiter.

10. Kit (60) selon la revendication 9, comprenant en outre des doigts (65) de préhension de tissu biologique, agencés pour être montés sur un automate de découpe de tissu et/ou des doigts (64) de préhension de tissu biologique, agencés pour être montés sur un automate de déplacement du tissu.

11. Kit (60) selon l'une des revendications 9 et 10, dans lequel le réceptacle (62) du tissu à traiter comprend des moyens (66) de positionnement du tissu.

12. Kit (60) selon l'une des revendications 9 à 11, dans lequel le réceptacle (62) est agencé pour séparer le tissu de fluides expulsés lors d'une centrifugation.

13. Kit (60) selon l'une des revendications 9 à 12, dans lequel les doigts (65) de préhension de tissu biologique, agencés pour être montés sur un automate de découpe de tissu, comprennent des moyens pour agripper un tissu biologique.

## Patentansprüche

1. Automatisiertes Verfahren zur Behandlung eines biologischen Gewebes zur Herstellung einer Gewebematrix für Allografts oder Xenografts, wobei die Abfolge der Schritte wie folgt ist:
a) das Gewebe wird in eine Behandlungslösung (6a) eingebracht,
b) das Gewebe wird unter Rühren in der Lösung behandelt (6b),
c) das Gewebe wird aus der Behandlungslösung entfernt (6c),
d) das Reaktionsgefäß wird geleert (6d),
wobei die Abfolge der Schritte bis zum Ende der Behandlung wiederholt wird, **dadurch gekennzeichnet, dass** alle Schritte aller Sequenzen auf automatisierte Weise in einer "klassifizierten" Kammer (2) und innerhalb eines einzigen Reaktionsgefäßes (40) durchgeführt werden, indem die Behandlungslösung im Reaktionsgefäß zwischen zwei Sequenzen gewechselt wird.

2. Verfahren zur automatisierten Herstellung einer Gewebematrix für Allotransplantationen oder Xenotransplantationen innerhalb eines einzigen "klassifizierten" Reaktionsgefäßes, bei dem nach der Entnahme eines biologischen Gewebes die Matrix in einem einzigen Gefäß hergestellt wird:
▪ das Gewebe klassifiziert wird,
▪ das Gewebe wird geschnitten (4),
▪ das Gewebe behandelt wird (6) und
▪ die erhaltene Gewebematrix verpackt wird(8),
wobei das Gewebe durch die Abfolge der folgenden Schritte behandelt wird, die bis zum Ende der Behandlung wiederholt werden:
a) Das Gewebe wird in eine Behandlungslösung eingeführt (6a),
b) Das Gewebe wird unter Rühren in der Lösung behandelt (6b),
c) das Gewebe wird aus der Behandlungslösung entfernt (6d),
d) das Reaktionsgefäß wird geleert (6d),
wobei alle Schritte aller Behandlungssequenzen in einem einzigen Reaktionsgefäß (40) durchgeführt werden, indem die Behandlungslösung im Reaktionsgefäß zwischen zwei Sequenzen gewechselt wird.

3. Verfahren nach Anspruch 2, das außerdem die folgenden Schritte umfasst:
▪ Zentrifugieren (4) des biologischen Gewebes, und/oder
▪ Gefriertrocknen (7) der Gewebematrix vor dem Verpacken.

4. Reaktionsgefäß (40) für die Durchführung des Verfahrens nach einem der Ansprüche 1 bis 3, umfassend:
▪ einen Behälter (41), der so angeordnet ist, dass er eine Behandlungslösung für das biologische Gewebe enthält, in das er eingeführt wird
▪ einen Rezeptor (42) für das biologische Gewebe und
▪ Rührmittel (46),
**dadurch gekennzeichnet, dass** der Rezeptor abnehmbar ist.

5. Reaktionsgefäß nach Anspruch 4, bei dem der abnehmbare Rezeptor (43) einen perforierten Stützboden für das biologische Gewebe umfasst.

6. Reaktionsgefäß nach einem der Ansprüche 4 und 5, bei dem die Rührvorrichtung (46) mit dem Behälter (42) verbunden ist.

7. Reaktionsgefäß (40) nach Anspruch 5 oder 6, bei dem das Rührmittel (46) mindestens eine Schaufel umfasst, die radial entlang einer Stange (42) angeordnet ist, deren Ende der perforierte Trägerboden (43) für das biologische Gewebe ist.

8. Reaktionsgefäß (40) nach Anspruch 7, bei dem der Behälter (41) so angeordnet ist, dass er zumindest den Teil der Stange (42) enthält, an dem die Klinge (46) und der Boden (43) angeordnet sind.

9. Ein Bausatz (60) mit einer Platte (61), die einen Träger darstellt:
▪ das Reaktionsgefäß (40) nach einem der Ansprüche 4 bis 8 und
▪ ein Behältnis (62) für das zu behandelnde Gewebe.

10. Der Bausatz (60) nach Anspruch 9 umfasst auch Finger (65) zum Greifen des biologischen Gewebes, die so angeordnet sind, dass sie an einem Gewebeschneideautomaten angebracht werden können, und/oder Finger (64) zum Greifen des biologischen Gewebes, die so angeordnet sind, dass sie an einem Gewebebewegungsautomaten angebracht werden können.

11. Kit (60) nach Anspruch 9 oder 10, bei dem die Aufnahme (62) für das zu behandelnde Gewebe die Mittel (66) zur Positionierung des Gewebes umfasst.

12. Kit (60) nach einem der Ansprüche 9 bis 11, bei dem das Gefäß (62) so angeordnet ist, dass es das Gewebe von den während einer Zentrifugation ausgeschiedenen Flüssigkeiten trennt.

13. Kit (60) nach einem der Ansprüche 9 bis 12, bei dem die Finger (65) zum Greifen des biologischen Gewebes, die so angeordnet sind, dass sie an einem Gewebeschneideautomaten angebracht werden können, die Mittel zum Greifen eines biologischen Gewebes umfassen.

## Claims

1. An automated process for the treatment of a biological tissue to produce a tissue matrix for allografts or xenografts, wherein the sequence of steps is as follows:
a) the tissue is introduced into a treatment solution (6a),
b) the tissue is treated in the solution, with stirring (6b),
c) the tissue is removed (6c) from the treatment solution,
d) the reaction vessel is emptied (6d),
wherein the sequence of steps is repeated till the end of the treatment, **characterised in that** all the steps of all the sequences are implemented in an automated manner in a "classified" chamber (2) and inside a single reaction vessel (40), by changing the treatment solution in the reaction vessel between two sequences.

2. A process for obtaining a tissue matrix for allografts or xenografts, in an automated manner inside a single "classified" reaction vessel, according to which, after collecting a biological tissue:
▪ the tissue is classified,
▪ the tissue is cut (4),
▪ the tissue is treated (6) and
▪ the obtained tissue matrix is packed (8),
wherein the tissue is treated by the sequence of the following steps, which are repeated till the end of the treatment:
a) the tissue is introduced (6a) into a treatment solution,
b) the tissue is treated in the solution, with stirring (6b),
c) the tissue is removed (6d) from the treatment solution,
d) the reaction vessel is emptied (6d),
with all the steps of all the sequences of the treatment being implemented inside a single reaction vessel (40) by changing the treatment solution in the reaction vessel between two sequences.

3. The process according to claim 2, further comprising the steps of:
▪ centrifuging (4) the biological tissue, and/or
▪ freeze-frying (7) the tissue matrix before packing.

4. A reaction vessel (40) for the implementing the process according to any of claims 1 to 3 comprising:
▪ a container (41) arranged to contain a treatment solution for the biological tissue into which is introduced
▪ a receptor (42) for the biological tissue and
▪ means of stirring (46),
**characterised in that** the receptor is detachable.

5. The reaction vessel according to claim 4, in which the detachable receptor (43) comprising a perforated support base for the biological tissue.

6. The reaction vessel according to claims 4 pr 5, in which the means of stirring (46) are connected to the receptor (42).

7. The reaction vessel (40) according to claims 5 or 6, in which the means of stirring (46) comprises at least one blade that is radially arranged along a rod (42), one end of which is the perforated support base (43) for the biological tissue.

8. The reaction vessel (40) according to claim 7, of which the container (41) is arranged to contain at least the part of the rod (42) on which the blade (46) and the base (43) are arranged.

9. A kit (60) comprising a plate (61) supporting:
▪ the reaction vessel (40) according to any one of claims 4 to 8 and
▪ a receptacle (62) for the tissue to be treated.

10. The kit (60) according to claim 9, also comprising fingers (65) for gripping the biological tissue, arranged to be mounted on a tissue cutting automaton and/or fingers (64) for gripping the biological tissue, arranged to be mounted on a tissue moving automaton.

11. The kit (60) according to claims 9 or 10, in which the receptacle (62) for the tissue to be treated includes the means (66) for positioning the tissue.

12. The kit (60) according to any one of claims 9 to 11, in which the receptacle (62) is arranged to separate the tissue from the fluids expelled during a centrifugation.

13. The kit (60) according to any one of claims 9 to 12, in which the fingers (65) for gripping the biological tissue, arranged to be mounted on a tissue cutting automaton, include the means to grip a biological tissue.
